# EUROPÄISCHE PATENTSCHRIFT

(11) **EP 1 475 367 B1**
(45) Veröffentlichungstag und Bekanntmachung des Hinweises auf die Patenterteilung: **07.07.2010**
(21) Anmeldenummer: 04009786.7
(22) Anmeldetag: 24.04.2004
(51) Int. Cl.: C07C 263/20, C07C 265/14

(54) **Verfahren zur Destillation eines Gemisches isomerer Diisocyanatodiphenylmethane**
Distillation process for separating diisocyanatodiphenylmethane isomers
Procédé de séparation par distillation de mélanges d'isomères de diisocyanato diphénylméthane

(30) Priorität: 08.05.2003 DE 10320504; 14.01.2004 DE 102004001872
(43) Veröffentlichungstag der Anmeldung: 10.11.2004
(73) Patentinhaber: Bayer MaterialScience AG, 51368 Leverkusen (DE)
(72) Erfinder: Schal, Hans-Peter, Dr., 41539 Dormagen (DE); Wolf, Ulrich, 47647 Kerken (DE); Thelen, Bernd, 51373 Leverkusen (DE)

(56) Entgegenhaltungen:
- EP-A- 0 079 516
- DE-A- 2 425 658

## Beschreibung

Die Erfindung betrifft ein Verfahren zur Destillation eines Gemisches wenigstens bestehend aus 2,2'-Diisocyanatodiphenylmethan, 2,4'-Diisocyanatodiphenylmethan und 4,4-Diisocyanatodiphenylmethan zur Gewinnung von 4,4'-Diisocyanatodiphenylmethan sowie von an 2,2'-Diisocyanatodiphenylmethan armen Gemischen aus 4,4'- und 2,4'-Düsocyanatodiphenylmethan.

Diisocyanatodiphenylmethan-Isomere sind Bestandteile von Polyisocyanatgemischen der Diphenylmethanreihe, welche bei der Phosgenierung von Anilin/Formaldehyd-Kondensaten, im folgenden auch Polyaminopolyphenylpolymethane genannt, entstehen.

Die Kondensation von Anilin und Formaldehyd sowie die Phosgenierung der Polyaminopolyphenylpolymethane ist aus dem Stand der Technik hinlänglich bekannt. Nach der Phosgenierung von Polyaminopolyphenylpolymethanen wird zunächst Phosgen vollständig entfernt. Dann werden die höheren Homologen des Diisocyanatodiphenylmethans (auch Polyisocyanatopolyphenylpolymethane genannt) abgetrennt. Aus dem verbleibenden Gemisch isomerer Diisocyanatodiphenylmethane, hauptsächlich bestehend aus 2,2'-Diisocyanatodiphenylmethan, 2,4'-Düsocyanatodiphenylmethan und 4,4'-Diisocyanatodiphenylmethan, wird anschließend reines 4,4'-Diisocyanatodiphenylmethan abgetrennt. Für die Abtrennung sind verschiedene Verfahren auf Basis einer Destillation oder Kristallisation oder einer Kombination aus Destillation und Kristallisation aus dem Stand der Technik bekannt.

Als ein Beispiel für die Gewinnung von 4,4'-Diisocyanatodiphenylmethan mit Hilfe eines Kristallisationsverfahrens sei DE-A-2 322 574 genannt. Nachteilig an den Kristallisationsverfahren ist ihr hoher Energiebedarf, da insbesondere für hohe Reinheiten des 4,4'-Diisocyanatodiphenylmethans große Mengen an Kälteenergie zur Verfügung gestellt werden müssen.

Ein Beispiel für Destillationsverfahren zur Abtrennung von 4,4'-Diisocyanatodiphenylmethan ist DE-A-2 631 168 und DE-A-24 25 658. Das Verfahren beschreibt die mehrstufige Aufarbeitung eines Gemisches aus Polyisocyanatopolyphenylpolymethanen zu Diisocyanatodiphenylmethan-Isomeren. Nach einer destillativen Abtrennung der höherfunktionellen Isocyanate, d.h. solcher mit mehr als 2 Isocyanatgruppen pro Molekül, wird der auf dieser Stufe anfallende erste Destillationsstrom, bestehend im Wesentlichen aus 2,2'-Diisocyanatodiphenylmethan (nachfolgend auch mit 2,2'-MDI abgekürzt), 2,4'-Düsocyanatodiphenylmethan (nachfolgend auch mit 2,4'-MDI abgekürzt) 4,4'-Düsocyanatodiphenylmethan (nachfolgend auch mit 4,4'-MDI abgekürzt) einer ersten Kolonne zugeführt und in einen weiteren Destillationsstrom und einen Sumpfstrom aufgetrennt. Der Sumpfstrom kann bis zu 10 Gew.-% des ersten Destillationsstroms betragen. Der zweite Destillationsstrom wird in einer zweiten Kolonne in einen Kopfstrom, welcher leichtflüchtige Verunreinigungen, 2,2'-Diisocyanatodiphenylmethan und 2,4'-Diisocyanatodiphenylmethan enthält, und einen Sumpfstrom, welcher die überwiegenden Anteile an 2,4'-MDI und 4,4'-Diisocyanatodiphenylmethan enthält, fraktioniert. Dieser Sumpfstrom wird in einer dritten Kolonne in 4,4'-MDI und einen Destillatanteil, angereichert mit 2,4'-MDI, aufgetrennt. In der letzten Destillationsstufe destilliert 4,4'-MDI mit einem Gehalt von weniger als 2 Gew.-% 2,4'-MDI.

Weitere Verfahren zur destillativen Gewinnung von 4,4'-Diisocyanatodiphenylmethan bzw. von Gemischen aus 4,4'- und 2,4'-Diisocyanatodiphenylmethan sind z.B. in DE-A-2 933 601 und DE-A-3 145 010 beschrieben. In DE-A-3 145 010 wird vorgeschlagen, dass aus dem Isomerengemisch der Diisocyanatodiphenylmethane zunächst als Kopfprodukt 2,2'- und 2,4'-Diisocyanatodiphenylmethan abgezogen werden und als Sumpfprodukt weitgehend von Isomeren befreites 4,4'-MDI erhalten wird. Dieses Sumpfprodukt ist dann in einer Enddestillation von Polymerisationsprodukten, die sich während der thermischen Belastung gebildet haben, zu befreien, während das Kopfprodukt einer weiteren destillativen Aufarbeitung zugeführt wird.

In gewöhnlichen Destillationskolonnen wird der Einsatzstrom üblicherweise in zwei Produktströme aufgetrennt: in ein Kopfprodukt und ein Sumpfprodukt. Die Fraktionierung eines Mehrkomponentenstroms gelingt daher nicht vollständig. Notwendige weitere Auftrennungen können beispielsweise dadurch vorgenommen werden, dass entweder der Sumpfstrom oder der Kopfstrom einem weiteren Destillationsschritt, ähnlich dem ersten, unterworfen wird. Gegebenenfalls können weitere Destillationsschritte angeschlossen werden. In kontinuierlichen Prozessen ist es dabei erforderlich, für jeden Destillationsschritt eine eigene Kolonne mit den zugehörigen Verdampfern und Kondensatoren bereitzustellen. Eine solche Abfolge von Destillationsschritten führt somit sowohl zu erheblichem apparativen Aufwand als auch zu erheblichem Energieaufwand. Die Betriebskosten eines solchen mehrstufigen Destillationsverfahrens sind entsprechend hoch. Ferner wird im Falle der MDI-Destillation durch die thermische Belastung über mehrere Destillationsstufen ein wesentlicher Rückstand gebildet, bestehend aus Nebenprodukten wie z.B. Uretdionen und Carbodiimiden, wodurch die Menge an Zielprodukt verringert wird.

Aufgabe der vorliegenden Erfindung ist es, ein Verfahren zur Destillation eines Gemisches isomerer Diisocyanatodiphenylmethane, wenigstens bestehend aus 2,2'-Diisocyanatodiphenylmethan, 2,4'- Diisocyanatodiphenylmethan und 4,4'-Diisocyanatodiphenylmethan, bereitzustellen, bei dem 4,4'-Diisocyanatodiphenylmethan in hoher Reinheit von mindestens 98 Gew.-% gewonnen wird. Das Destillationsverfahren soll einen geringeren apparativen Aufwand sowie einen geringeren Energieaufwand erfordern als herkömmliche Destillationsverfahren zur Trennung isomerer Düsocyanatodiphenylmethane.

Gegenstand der Erfindung ist ein Verfahren zur Destillation eines Gemisches isomerer Düsocyanatodiphenylmethane, wenigstens bestehend aus 2,2'-Diisocyanatodiphenylmethan, 2,4'-Diisocyanatodiphenylmethan und 4,4'-Diisocyanatodiphenylmethan, wobei die Destillation mindestens einstufig ausgeführt ist und in mindestens einer Destillationsstufe eine Trennwandkolonne eingesetzt wird.

Die Destillation eines Mehrkomponentengemisches in einer Trennwandkolonne ist beispielsweise aus der Lehre von US 2 471 134 bekannt. In einer Trennwandkolonne verläuft eine Trennwand vertikal im mittleren Teil der Kolonne. Dadurch wird die Kolonne in vier Zonen unterteilt: eine Vorfraktionierungszone und eine Hauptfraktionierungszone im Bereich der Trennwand sowie eine Sumpfzone (Abtriebszone) und eine Rektifizierzone (Kopfzone). In die Vorfraktionierungszone wird der Mehrkomponentenstrom eingespeist. Das Kopfprodukt wird der Rektifizierzone, das Sumpfprodukt der Abtriebszone entnommen. Der Hauptfraktionierungszone wird ein intermediäres Produkt entnommen.

Nach dem erfindungsgemäßen Verfahren können Diisocyanatodiphenylmethangemische (im folgenden auch als Ausgangsgemische bezeichnet) mit unterschiedlichsten Anteilen an 2,2'-Diisocyanatodiphenylmethan, 2,4'-Diisocyanatodiphenylmethan und 4,4'-Diisocyanatodiphenylmethan destilliert werden. Die Anteile an 4,4'-MDI betragen bevorzugt von 35 bis 95 Gew.-%, die Summe aus 2,2'-MDI und 2,4'-MDI beträgt bevorzugt von 5 bis 65 Gew.-%. Der Anteil an 2,2'-MDI beträgt bevorzugt von 1 bis 10 Gew.-% bezogen auf 2,4'-MDI.

Vorzugsweise werden Gemische umfassend maximal 3,0 Gew.-% 2,2'-Diisocyanatodiphenylmethan, 5 bis 50 Gew.-% 2,4'-Diisocyanatodiphenylmethan und 50 bis 95 Gew.-% 4,4'-Diisocyanatodiphenylmethan destilliert. Besonders bevorzugt sind Gemische bestehend aus 5 bis 25 Gew.-% 2,4'-MDI und 75 bis 95 Gew.-% 4,4'-MDI.

Darüber hinaus kann das Ausgangsgemisch enthalten: Chlorbenzol und andere leichter siedende Verbindungen, z.B. Phenylisocyanat, mit einem Anteil von weniger als 2 Gew.-%, 0 bis 5 Gew.-% Polyisocyanatopolyphenylmethane sowie 0 bis 5 Gew.-% höher molekulare Verbindungen, die aufgrund thermischer Belastung entstanden sind.

Ein solches Gemisch isomerer Diisocyanatodiphenylmethane entsteht bei der Phosgenierung von Polyaminopolyphenylpolymethanen, welche durch Kondensation von Anilin und Formaldehyd hergestellt werden, zu Polyisocyanatopolyphenylpolymethanen. Nach der Phosgenierung, welche vorzugsweise in Monochlorbenzol (MCB) als Lösungsmittel durchgeführt wird, werden auf destillativem Wege zunächst das Lösungsmittel und Phosgen vollständig entfernt. Dann wird mittels Destillation in einer an sich bekannten, z.B. aus DE 2 631 168, Polymerabtrennung ein Gemisch aus Polyisocyanatopolyphenylpolymethanen und Diisocyanatodiphenylmethanen einerseits und ein Gemisch der drei Isomeren 2,2'-Diisocyanatodiphenylmethan, 2,4'-Diisocyanatodiphenylmethan und 4,4'-Diisocyanatodiphenylmethan neben Resten an Lösungsmittel und anderen Leichtsiedern wie Phenylisocyanat andererseits erhalten. Letzteres dient als Ausgangsgemisch für das erfindungsgemäße Verfahren.

Das Ausgangsgemisch isomerer Diisocyanatodiphenylmethane wird der Trennwandkolonne seitlich im Bereich der Trennwand zugeführt.

Die Trennwandkolonne befindet sich im mittleren Bereich der Kolonne. Die Länge der Trennwand ist abhängig von den Prozessbedingungen und von den Eigenschaften der eingesetzten Stoffaustauschelemente. Bei dem Einsatz einer Gewebepackung mit z.B. 500 m²/m³ spezifischer Oberfläche beträgt die Länge der Trennwand ca. 8 m, d.h. 2/3 der gesamten Stoffaustauschzone liegen im Bereich der Trennwand. Die Trennwand teilt die Kolonne in eine Vorfraktionierungszone und eine Hauptfraktionierungszone.

Die Dampfströmung in der Vorfraktionierungszone und der Hauptfraktionierungszone stellt sich entsprechend den Packungsdruckverlusten ein. Der Gesamtdruck an den Ein- und Austrittsbereichen der Trennwand ist für beide Zonen gleich. Falls aus prozesstechnischer Sicht im Bereich der Trennwand eine Zone stärker mit Dampf beaufschlagt werden soll, können die Querschnitte von Vorfraktionierungszone und Hauptfraktionierungszone auch unterschiedlich gewählt werden. Durch entsprechende Wahl der Teilquerschnitte der beiden Zonen kann der Prozess optimiert werden.

Als Stoffaustauschelemente sind Packungen besonders geeignet. Verwendbar sind aber auch andere in der Destillationstechnik bekannte Elemente, wie z.B. Füllkörper oder Böden.

Die Trennwandkolonne wird hinsichtlich Druck und Temperatur unter ähnlichen Prozessbedingungen betrieben wie eine Kolonne der konventionellen Destillationstechnik. Der Kopfdruck liegt vorzugsweise im Bereich von 3 bis 12 mbar. Je nach Gemischzusammensetzung beträgt die Kopftemperatur bevorzugt 165 bis 200°C. Der Sumpfdruck beträgt vorzugsweise von 11 bis 20 mbar bei bevorzugten Temperaturen von 210 bis 225°C.

Bei der Destillation des Gemisches isomerer Diisocyanatodiphenylmethane in der Trennwandkolonne wird als Sumpfprodukt 4,4'-Diisocyanatodiphenylmethan mit einer Isomerenreinheit, d.h. einer Reinheit bezogen auf die drei Isomere 2,2'-MDI, 2,4'-MDI und 4,4'-MDI, von mindestens 98 Gew.-% erhalten.

Bei der einstufigen Destillation des Gemisches isomerer Diisocyanatodiphenylmethane in der Trennwandkolonne wird vorzugsweise als Sumpfprodukt 4,4'-Diisocyanatodiphenylmethan mit einer Reinheit von mindestens 98 Gew.-%, als Kopfprodukt in Abhängigkeit von dem Feedstrom ein Gemisch, bestehend aus 2,2'-Diisocyanatodiphenylmethan von max. 60 Gew.-%, 2,4'-Diisocyanatodiphenylmethan von 40 bis 80 Gew.-% und 4,4'-Diisocyanatodiphenylmethan bis 5 Gew.-% sowie als Seitenstrom ein Gemisch aus 2,4'-Diisocyanatodiphenylmethan und 4,4'-Diisocyanatodiphenylmethan mit einem Gewichtsverhältnis von 85:15 bis 15:85 erhalten.

Besonders bevorzugt wird die Trennwandsektion so ausgeführt, dass im Seitenstrom ein MDI-Gemisch von 50 bis 60 Gew.-% 2,4'-MDI und 40 bis 50 Gew.-% 4,4'-MDI abgezogen wird. Durch geeignete Flüssigkeitsaufteilung zwischen Trennwand und Rektifizierzone kann die Konzentration von 2,4'-MDI im Seitenstrom in weiten Grenzen von 15 bis 85 Gew.-% beeinflusst werden.

Das Rücklaufverhältnis im Kopf wird insbesondere im Bereich von 10 bis 250 eingestellt, liegt jedoch besonders bevorzugt im Bereich von 60 bis 120, wobei der Destillatstrom 1 bis 5 Gew.-% bezogen auf den Feedstrom beträgt. Der Sumpfstrom beträgt 60 bis 90 Gew.-%, vorzugsweise 75 bis 85 Gew.-% des Feedstromes.

Alternativ kann das erfindungsgemäße Destillationsverfahren auch zweistufig ausgeführt sein, wobei die erste Destillationsstufe in einer Destillationskolonne ohne Trennwand, die zweite Stufe mit Trennwandkolonne durchgeführt wird. Dabei wird das Kopfprodukt der ersten Destillationsstufe der Trennwandkolonne der zweiten Stufe zugeführt.

Im zweistufigen Verfahren wird die zweite Destillationsstufe mit Trennwandkolonne unter vergleichbaren Bedingungen wie im einstufigen Verfahren durchgeführt. Die Trennwandkolonne wird hinsichtlich Druck und Temperatur unter ähnlichen Prozessbedingungen betrieben wie eine Kolonne der konventionellen Destillationstechnik. Der Kopfdruck liegt im Bereich von 3 bis 12 mbar. Je nach Zusammensetzung des Gemisches beträgt die Kopftemperatur 165 bis 200°C. Der Sumpfdruck liegt im Bereich von 11 und 20 mbar bei Temperaturen von 210 bis 225°C. Die Trennwandsektion wird so ausgeführt, dass im Seitenstrom vorzugsweise ein MDI-Gemisch von 50 bis 60 Gew.-% 2,4'-MDI und 40 bis 50 Gew.-% 4,4'-MDI abgezogen werden kann. Durch geeignete Flüssigkeitsaufteilung zwischen Trennwand und Rektifizierzone kann die Konzentration von 2,4'-MDI im Seitenstrom in weiten Grenzen von 15 bis 85 Gew.-% beeinflusst werden. Das Rücklaufverhältnis im Kopf wird bevorzugt im Bereich von 5 bis 80 eingestellt, besonders bevorzugt im Bereich von 10 bis 40, wobei der Destillatstrom 5-20 Gew.-% bezogen auf den Feedstrom beträgt. Der Sumpfstrom beträgt vorzugsweise von 7 bis 30 Gew.-%, besonders bevorzugt von 15 bis 25 Gew.-% des Feedstromes.

Um eine noch höhere Reinheit des 4,4'-MDI zu erzielen, wird in einer bevorzugten Ausführungsform des erfindungsgemäßen Verfahrens nach der Destillation in der Trennwandkolonne eine zusätzliche Destillation des Sumpfproduktes der Trennwandkolonne in einer Destillationskolonne ohne Trennwand durchgeführt. Diese zusätzliche Destillation zur Erreichung einer höheren Reinheit kann sowohl bei dem einstufigen Verfahren mit einer Trennwandkolonne als auch bei dem zweistufigen Verfahren mit einer ersten Destillationskolonne ohne Trennwand und einer zweiten Destillationskolonne mit Trennwand eingesetzt werden.

In einer weiteren Ausführungsform ist die Destillation des Gemisches isomerer Diisocyanatodiphenylmethane zweistufig, wobei in jeder Destillationsstufe eine Trennwandkolonne eingesetzt wird. Das Kopfprodukt der ersten Trennwandkolonne wird der zweiten Trennwandkolonne zugeführt. Durch die Kombination zweier Trennwandkolonnen entfällt die der Destillation sonst üblicherweise nachgeschaltete Reinigungsstufe in einer herkömmlichen Destillationskolonne, wie oben beschrieben, zur Erreichung besonders hoher Reinheiten.

Aus der ersten Trennwandkolonne wird kommerziell verwendbares 4,4'-MDI mit vorzugsweise mindestens 98 Gew.-%, besonders bevorzugt 98,5 bis 99,0 Gew.-%, als Seitenstrom entnommen. Das Kopfprodukt der ersten Kolonne enthält im Wesentlichen das gesamte zugeführte 2,2'-MDI und besteht aus einem Isomerengemisch aus 2,2'-MDI (vorzugsweise 0,5 bis 5,0 Gew.-%), 2,4'-MDI (29,0 bis 55,0 Gew.-%) und 4,4'-MDI (40,0 bis 70,0 Gew.-%). Außerdem enthält es die mit dem Feed zugeführten Leichtsiederkomponenten wie z.B. Chlorbenzol. Der Sumpf enthält überwiegend 4,4'-MDI (99,5 bis 99,95 Gew.-%) mit höhermolekularen Nebenprodukten als Verunreinigungen, welche aufgrund der thermischen Belastung entstanden sind, und weniger als 0,5 Gew.-% 2,4' MDI.

Die erste Trennwandkolonne wird hinsichtlich Druck und Temperatur unter ähnlichen Prozessbedingungen betrieben wie eine Kolonne der konventionellen Destillationstechnik. Der Kopfdruck liegt bevorzugt im Bereich von 3 bis 12 mbar. Je nach Gemischzusammensetzung beträgt die Kopftemperatur vorzugsweise 165 bis 200°C. Der Sumpfdruck beträgt bevorzugt 11 und 20 mbar bei Temperaturen von bevorzugt 210 bis 225°C. Die ablaufende Flüssigkeit aus der Rektifizierzone wird zu 50 bis 80 Gew.-% auf die Vorfraktionierzone gegeben. Die restlichen 20 bis 50 Gew.-% werden zur Hauptfraktionierzone geleitet. Im Seitenstrom werden 40 bis 80 Gew.-% als Produktstrom abgezogen.

Die zweite Trennwandkolonne in dieser Ausführungsform des erfindungsgemäßen Verfahrens wird unter vergleichbaren Bedingungen wie die Trennwandkolonne der zweiten Stufe im oben beschriebenen zweistufigen Verfahren mit herkömmlicher Destillationskolonne in der ersten Destillationsstufe betrieben. Die Zusammensetzung des Kopfstromes dieser zweiten Trennwandkolonne entspricht im Wesentlichen der Zusammensetzung des Kopfstromes der Trennwandkolonne im einstufigen Verfahren, d.h. maximal 60 Gew.-% 2,2' MDI, 40 bis 80 Gew.-% 2,4' MDI und maximal 5 Gew.-% 4,4' MDI. Der Seitenstrom enthält maximal 0,2 Gew.-% 2,2' MDI, 50 bis 60 Gew.-% 2,4' MDI und 40 bis 50 Gew.-% 4,4' MDI. Der Sumpfstrom ist im Wesentlichen frei von 2,2'-MDI. Die Konzentration von 2,4'-MDI kann bis zu 25 Gew.-% betragen, beträgt jedoch bevorzugt kleiner als 2 Gew.-%. Somit kann auch dieses Produkt kommerziell verwendet werden.

Der Einsatz mindestens einer Trennwandkolonne zur Gewinnung von 4,4'-MDI in hoher (mindestens 98 Gew.-%) bzw. sehr hoher Reinheit (mindestens 99 Gew.-%) aus einem Gemisch dreier isomerer Diisocyanatodiphenylmethane ermöglicht es, im Vergleich zu Destillationsverfahren nach dem Stand der Technik eine bzw. zwei Destillationsstufen einzusparen. Damit ist einerseits der apparative Aufwand erheblich geringer, da neben der Destillationskolonne auch zusätzliche Wärmetauscher, Verrohrungen, usw. eingespart werden. Andererseits reduziert sich dadurch der Energieaufwand in bedeutendem Maße, da weniger Wärme zugeführt werden muss. Des Weiteren ist bei einer geringeren Anzahl von Destillationsstufen aufgrund der geringeren thermischen Belastung der Rückstandsanteil, bedingt durch Reaktionen der Isocyanatgruppen, geringer.

Nachfolgend wird die Erfindung anhand der Zeichnungen und der Beispiele näher erläutert. Es zeigen:
Figur 1 ein Schema einer ersten Ausführungsform des erfindungsgemäßen Verfahrens mit einer Trennwandkolonne
Figur 2 ein Schema einer zweiten Ausführungsform des erfindungsgemäßen Verfahrens, bei dem in der ersten Stufe eine herkömmliche Destillationskolonne und in der zweiten Stufe eine Trennwandkolonne eingesetzt wird
Figur 3 ein Schema einer weiteren Ausführungsform des erfindungsgemäßen Verfahrens mit zwei Destillationsstufen, wobei in jeder Destillationsstufe eine Trennwandkolonne eingesetzt wird.

In den Figuren sind gleiche oder ähnliche Destillationskolonnen und Ströme mit gleichen Bezugszeichen versehen.

### Beispiele:

### Beispiel 1:

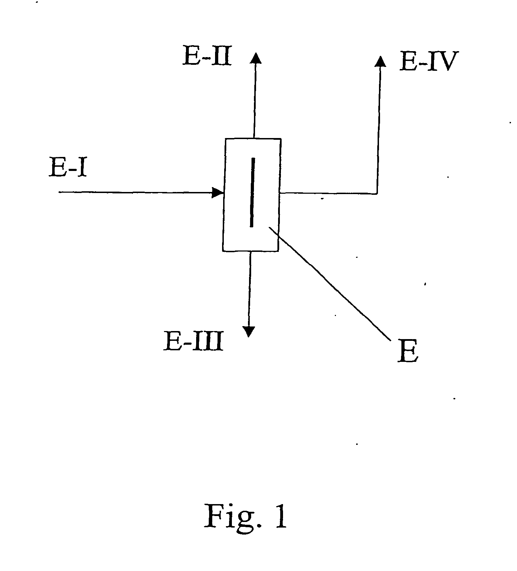

Es wird eine einstufige Destillation mit einer Trennwandkolonne gemäß Figur 1 durchgeführt. 5,9 kg/h eines Isomerengemisches, bestehend aus 0,6 Gew.-% 2,2'-MDI, 11,1 Gew.-% 2,4'-MDI und 88,3 Gew.-% 4,4'-MDI werden der Trennwandkolonne E im Bereich der Trennwand zugeführt. Der Trennwandkolonne werden drei Produktströme entnommen: 0,15 kg/h Kopfstrom E-II, bestehend aus 23,5 Gew.-% 2,2'-MDI, 75,0 Gew.-% 2,4'-MDI und 1,5 Gew.-% 4,4'-MDI und 0,95 kg/h Seitenstrom E-IV, bestehend aus 0,05 Gew.-% 2,2'-MDI, 54,90 Gew.-% 2,4'-MDI, 44,05 Gew.-% 4,4'-MDI sowie 4,8 kg/h Sumpfstrom E-III mit einer Isomerenreinheit an 4,4'-MDI von 99 %. Zur Erhöhung der Reinheit des 4,4'-MDI kann der Sumpfstrom E-III in eine zusätzliche Destillationskolonne ohne Trennwand überführt werden.

Als Stoffaustauschelemente werden in der Trennwandkolonne Gewebepackungen mit 500 m²/m³ spezifischer Oberfläche eingesetzt. 67 Gew.-% der Flüssigkeit wird auf die Vorfraktionierzone und 33 Gew.-% auf die Hauptfraktionierzone geleitet. Die Rektifizierzone und die Abtriebszone besitzen jeweils 8 Trennstufen, die Vorfraktionierungszone und die Hauptfraktionierungszone jeweils 12 Trennstufen oben und unten, wobei mit den Trennstufen oben und unten - hier und im folgenden - die Trennstufen oberhalb und unterhalb der Zuführung des Feedstromes in die Vorfraktionierungszone bzw. der Seitenstromentnahme aus der Hauptfraktionierungszone gemeint ist. Der Kopfdruck beträgt 6 mbar. Der Rücklauf an der Destillatentnahme beträgt 90:1, der Rücklauf an der Seitenstromentnahme beträgt 2,6:1.

### Beispiel 2:

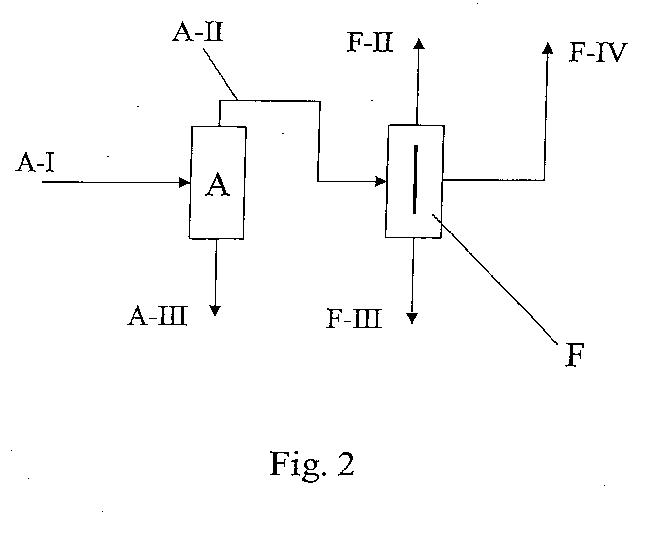

Es wird eine zweistufige Destillation mit einer gewöhnlichen Destillationskolonne A in Stufe 1 und einer Trennwandkolonne F in Stufe 2 gemäß Figur 2 durchgeführt.

Gemäß Figur 2 werden 6,3 kg/h eines Isomerengemisches, bestehend aus 2,2'-MDI (0,6 Gew.-%), 2,4'-MDI (11,1 Gew.-%) und 4,4'-MDI (88,3 Gew.-%), der Rektifikationskolonne A zugeführt (Strom A-I). Am Kopf der Rektifikationskolonne A werden 1,55 kg/h mit 2,3 Gew.-% 2,2'-MDI, 42,0 Gew.-% 2,4'-MDI und 55,7 Gew.-% 4,4'-MDI abgezogen (Strom A-II). Das Sumpfprodukt der Kolonne A (Strom A-III, 4,8 kg/h) enthält 4,4'-MDI von hoher Reinheit (97 Gew.-%). Der Kopfdruck in der Kolonne A beträgt 6 mbar. Der Rücklauf an der Destillatentnahme beträgt 6,5: 1. Die Rektifizierzone weist 8 Trennstufen auf, die Abtriebszone 18 Trennstufen.

Der Kopfstrom A-II wird in die Trennwandkolonne F eingespeist. Bei der Destillation werden 0,15 kg/h eines Kopfstroms bestehend aus 24,5 Gew.-% 2,2'-MDI, 75,3 Gew.-% 2,4'-MDI und 0,2 Gew.-% 4,4'-MDI entnommen (Strom F-II). Am Sumpf der Kolonne (Strom F-III) wird 4,4'-MDI mit einer Isomerenereinheit von 99% entnommen (0,4 kg/h). Der Seitenstrom F-IV, der aus dem unteren Viertel der Trennwandzone entnommen wird, besteht aus weniger als 0,1 Gew.-% 2,2'-MDI, 55 Gew.-% 2,4'-MDI und 45 Gew.-% 4,4'-MDI. In der Trennwandkolonne F beträgt der Kopfdruck 6 mbar. Der Rücklauf an der Destillatentnahme beträgt 21:1, an der Seitenstromentnahme 1,4: 1. Die Rektifizierzone sowie die Sumpfzone weisen jeweils 8 Trennstufen auf. Die Vorfraktionierungszone besitzt 8 Trennstufen oben und 20 Trennstufen unten. Die Hauptfraktionierungszone weist 22 Trennstufen oben und 6 Trennstufen unten auf. Als Stoffaustauschelemente werden in der Trennwandkolonne F Gewebepackungen mit 500 m²/m³ spezifischer Oberfläche eingesetzt. 67 Gew.-% der Flüssigkeit werden auf die Vorfraktionierzone und 33 Gew.-% auf die Hauptfraktionierzone geleitet.

Zur Erzielung einer höheren Reinheit von 4,4'-MDI können die Ströme A-III und/oder F-III in eine zusätzliche Destillationskolonne ohne Trennwand, beispielsweise zur Flashdestillation, überführt werden.

### Beispiel 3:

Es wird eine einstufige Destillation mit einer Trennwandkolonne E gemäß Figur 1 durchgeführt. Der Trennwandkolonne E werden 6,33 kg/h eines Isomerengemisches, bestehend aus 0,6 Gew.-% 2,2'-MDI, 10,8 Gew.-% 2,4'-MDI, 87,9 Gew.-% 4,4'-MDI und 0,7 Gew.-% höher siedender Verunreinigungen, zugeführt (Feedstrom E-I). Der Sumpfstrom E-III beträgt 0,4 kg/h und besteht aus 99 Gew.-% 4,4'-MDI und 1 Gew.-% Verunreinigungen. Der Kopfstrom E-II ist ein Isomerengemisch, bestehend aus 2,4 Gew.-% 2,2'-MDI, 41,0 Gew.-% 2,4'-MDI und 56,6 Gew.-% 4,4'-MDI. Die Reinheit von 4,4'-MDI, welches als Seitenstrom E-IV mit 4,37 kg/h entnommen wird, beträgt 98,9 Gew.-%.

Als Stoffaustauschelemente werden in der Trennwandkolonne E Gewebepackungen mit 500 m²/m³ spezifischer Oberfläche eingesetzt. 67 Gew.-% der Flüssigkeit werden auf die Vorfraktionierzone und 33 Gew.-% auf die Hauptfraktionierzone geleitet. Der Kopfdruck beträgt 6 mbar. Der Rücklauf an der Destillatentnahme beträgt 6,5:1, der Rücklauf an der Seitenstromentnahme 1,8:1. Die Rektifizierzone weist 8 Trennstufen auf, die Abtriebszone maximal 4 Trennstufen. Die Vorfraktionierungszone besitzt 8 Trennstufen oben und 16 Trennstufen unten. Die Hauptfraktionierungszone besitzt 16 Trennstufen oben und 8 Trennstufen unten.

Der Kopfstrom E-II (Fig. 1) kann zur weiteren Aufarbeitung entweder einer gewöhnlichen Destillationskolonne oder einer Trennwandkolonne zugeführt werden, deren Sumpfstrom mit einem geringen Anteil an 2,2'-MDI wiederum einer weiteren gewöhnlichen Destillationskolonne zugeführt werden kann. Am Sumpf dieser zweiten gewöhnlichen Destillationskolonne wird reines 4,4'-MDI erhalten, der Kopfstrom liefert ein 2,4'-MDI reiches Produkt.

### Beispiel 4:

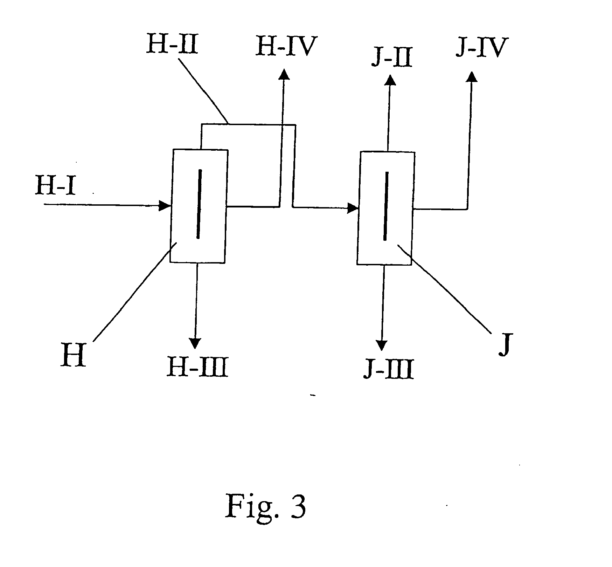

Es wird eine zweistufige Destillation mit zwei Trennwandkolonnen H und J gemäß Fig. 3 durchgeführt. Dabei wird das Isomerengemisch mit einer Zusammensetzung von 0,5 Gew.-% 2,2'-MDI, 11,1 Gew.-% 2,4'-MDI und 88,4 Gew.-% 4,4'-MDI auf die erste Kolonne H im Bereich der Trennwand geführt (Strom H-I, 6,33 kg/h). Als Sumpfprodukt H-III (0,4 kg/h) wird ein hoch angereichertes 4,4'-MDI (Isomerenreinheit 99 %) mit Verunreinigungen erhalten. Der Gehalt an 4,4'-MDI in der Seitenstromentnahme H-N (4,38 kg/h) beträgt 98,8 Gew.-%. Mit H-IV werden 77 Gew.-% des eingespeisten 4,4'-MDI abgeführt.

Als Stoffaustauschelemente werden in der Trennwandkolonne H Gewebepackungen mit 500 m²/m³ spezifischer Oberfläche eingesetzt. 67 Gew.-% der Flüssigkeit werden auf die Vorfraktionierzone und 33 Gew.-% auf die Hauptfraktionierzone geleitet. Der Kopfdruck beträgt 6 mbar. Der Rücklauf an der Destillatentnahme beträgt 6,5:1, der Rücklauf an der Seitenstromentnahme 1,8:1. Die Rektifizierzone weist 8 Trennstufen auf, die Abtriebszone maximal 4 Trennstufen. Die Vorfraktionierungszone besitzt 8 Trennstufen oben und 16 Trennstufen unten. Die Hauptfraktionierungszone besitzt 16 Trennstufen oben und 8 Trennstufen unten.

Das Kopfprodukt H-II ist ein mit ortho-ständigen Isocyanatgruppen angereichertes Isomerengemisch, dass zur Herstellung 2,4'-MDI-reicher Produkte noch weiter aufgetrennt werden muss. Das Kopfprodukt H-II der ersten Kolonne H wird daher zur zweiten Trennwandkolonne J im Bereich der Trennwand geführt.

Als Sumpfprodukt J-III der Kolonne J wird ein von 2,2'-MDI befreites 4,4'-MDI (0,43 kg/h) mit 1 Gew.-% 2,4'-MDI bezogen auf den Isomerenanteil erhalten. Der Seitenstrom J-IV besitzt eine Zusammensetzung von 0,05 Gew.-% 2,2'-MDI, 55 Gew.-% 2,4'-MDI und 45 Gew.-% 4,4'-MDI. Mit dem Kopfprodukt (Strom J-II, 0,15 kg/h) der Zusammensetzung 23,5 Gew.-% 2,2'-MDI, 75 Gew.-% 2,4'-MDI und 1,5 Gew.-% 4,4'-MDI wird das 2,2'-MDI aus dem System herausgeführt.

In der Trennwandkolonne J beträgt der Kopfdruck 6 mbar. Der Rücklauf an der Destillatentnahme beträgt 21:1, an der Seitenstromentnahme 1,4:1. Die Rektifizierzone sowie die Sumpfzone weisen jeweils 8 Trennstufen auf. Die Vorfraktionierungszone besitzt 8 Trennstufen oben und 20 Trennstufen unten. Die Hauptfraktionierungszone weist 22 Trennstufen oben und 6 Trennstufen unten auf. Als Stoffaustauschelemente werden in der Trennwandkolonne J Gewebepackungen mit 500 m²/m³ spezifischer Oberfläche eingesetzt. 67 Gew.-% der Flüssigkeit werden auf die Vorfraktionierzone und 33 Gew.-% auf die Hauptfraktionierzone geleitet.

## Patentansprüche

1. Verfahren zur Destillation eines Gemisches isomerer Diisocyanatodiphenylmethane, wenigstens bestehend aus 2,2'-Diisocyanatodiphenylmethan, 2,4'-Diisocyanatodiphenylmethan und 4,4'-Diisocyanatodiphenylmethan, **dadurch gekennzeichnet, dass** die Destillation mindestens einstufig ausgeführt ist und in mindestens einer Destillationsstufe eine Trennwandkolonne eingesetzt wird.

2. Verfahren nach Anspruch 1, **dadurch gekennzeichnet, dass** das Gemisch isomerer Diisocyanatodiphenylmethane einen Anteil von 75 bis 95 Gew.-% 4,4'-Diisocyanatodiphenylmethan enthält.

3. Verfahren nach einem der Ansprüche 1 oder 2, **dadurch gekennzeichnet, dass** die Destillation zweistufig ausgeführt ist mit einer Trennwandkolonne in der zweiten Destillationsstufe, wobei das Kopfprodukt der ersten Destillationsstufe der Trennwandkolonne der zweiten Destillationsstufe zugeführt wird.

4. Verfahren nach einem der Ansprüche 1 bis 3, **dadurch gekennzeichnet, dass** nach der Destillation in der Trennwandkolonne das Sumpfprodukt der Trennwandkolonne einer zusätzlichen Destillation unterworfen wird.

5. Verfahren nach einem der Ansprüche 1 bis 4, **dadurch gekennzeichnet, dass** die Destillation zweistufig ausgeführt ist und in jeder Destillationsstufe eine Trennwandkolonne eingesetzt wird, wobei das Kopfprodukt der ersten Trennwandkolonne der zweiten Trennwandkolonne zugeführt wird.

## Claims

1. A process for the distillation of a mixture of isomeric diisocyanatodiphenylmethanes, at least consisting of 2,2'-diisocyanatodiphenylmethane, 2,4'-diisocyanatodiphenylmethane and 4,4'-diisocyanatodiphenylmethane, **characterised in that** the distillation is performed in at least one stage and a divided-wall column is used in at least one distillation stage.

2. A process according to claim 1, **characterised in that** the mixture of isomeric diisocyanatodiphenylmethanes contains a proportion of 75 to 95 % by weight 4,4'-diisocyanatodiphenylmethane.

3. A process according to one of claims 1 or 2, **characterised in that** the distillation is performed in two stages with a divided-wall column in the second distillation stage, wherein the overhead product from the first distillation stage is introduced into the divided-wall column of the second distillation stage.

4. A process according to any of claims 1 to 3, **characterised in that,** after the distillation in the divided-wall column, the bottoms product from the divided-wall column is subjected to an additional distillation.

5. A process according to any of claims 1 to 4, **characterised in that** the distillation is performed in two stages and a divided-wall column is used in each distillation stage, wherein the overhead product from the first divided-wall column is introduced into the second divided-wall column.

## Revendications

1. Procédé de distillation d'un mélange de diisocyanatodiphénylméthanes isomères, consistant au moins en le 2,2'-diisocyanatodiphénylméthane, le 2,4'-diisocyanatodiphénylméthane et le 4,4'-diisocyanatodiphénylméthane, **caractérisé en ce que** la distillation est réalisée en au moins une étape et **en ce que** dans au moins une étape de distillation, une colonne à paroi de séparation est mise en oeuvre.

2. Procédé selon la revendication 1, **caractérisé en ce que** le mélange de diisocyanatodiphénylméthanes isomères contient une proportion allant de 75 à 95% en poids de 4,4'-diisocyanatodiphénylméthane.

3. Procédé selon la revendication 1 ou 2, **caractérisé en ce que** la distillation est réalisée en deux étapes avec une colonne à paroi de séparation dans la deuxième étape de distillation, le produit de tête de la première étape de distillation étant alimenté dans la colonne à paroi de séparation de la deuxième étape de distillation.

4. Procédé selon l'une des revendications 1 à 3, **caractérisé en ce qu'**après la distillation dans la colonne à paroi de distillation, le produit de fond de la colonne à paroi de séparation est soumis à une distillation supplémentaire.

5. Procédé selon l'une des revendications 1 à 4, **caractérisé en ce que** la distillation est réalisée en deux étapes et que dans chaque étape de distillation, une colonne à paroi de séparation est mise en oeuvre, le produit de tête de la première colonne à paroi de séparation étant alimenté dans la deuxième colonne à paroi de séparation.
